# EUROPEAN PATENT SPECIFICATION

(11) **EP 4 005 467 B1**
(45) Date of publication and mention of the grant of the patent: **01.01.2025**
(21) Application number: 21210175.2
(22) Date of filing: 24.11.2021
(51) Int. Cl.: A61B 5/00

(54) **ACQUISITION GUIDANCE FOR ELECTROANATOMICAL MAPPING**
AUFNAHMEFÜHRUNG FÜR ELEKTROANATOMISCHE ABBILDUNG
GUIDAGE D'ACQUISITION DE CARTOGRAPHIE ÉLECTRO-ANATOMIQUE

(30) Priority: 25.11.2020 US 202017104033
(43) Date of publication of application: 01.06.2022
(73) Proprietor: Biosense Webster (Israel) Ltd, Yokneam, 2066717 (IL)
(72) Inventor: PALTI, Yair, 2066717 Yokneam (IL); GLINER, Vadim, 2066717 Yokneam (IL); ATTIAS, Gili, 2066717 Yokneam (IL); WEKSELMAN, Guy, 2066717 Yokneam (IL); ZILBERMAN, Israel, 2066717 Yokneam (IL)
(74) Representative: Carpmaels & Ransford LLP

(56) References cited:
- EP-A1- 3 723 098
- EP-A2- 2 662 049
- US-A1- 2018 296 108
- US-A1- 2019 269 367

## Description

### FIELD OF THE INVENTION

The present invention relates generally to cardiac electrophysiological (EP) mapping, and particularly to EP measurement systems which enhance operator interaction.

### BACKGROUND

In cardiac electroanatomical mapping systems that are known in the art, an operator - typically a physician - inserts a catheter through a patient's vascular system into a chamber of the heart. An electrode or electrode assembly at the distal end of the catheter contacts the myocardial tissue in the chamber and receives electrical signals from the tissue, which are conveyed through the catheter to a mapping console. The operator manipulates the catheter within the heart in order to acquire signals from many points within the heart chamber, thus enabling the console to construct a map showing the physical structure of the walls of the heart chamber and the distribution of electrical activity over the walls.

As the operator cannot see the distal end of the catheter in the heart chamber, a number of techniques have been developed to assist the operator in visualizing and understanding the process of EP signal acquisition. For example, U.S. Patent No. 10,617,317 describes a method for highlighting an electrode image according to an electrode signal. A graphical image of a heart of a patient is presented on a display screen, including icons representing a catheter that is positioned within the heart and an electrode on the catheter, while the electrode is in contact with tissue at a location in the heart. The method further includes acquiring, using the electrode, electrical signals from the tissue at the location, and processing the acquired signals so as to detect an occurrence of a predefined signal feature in the acquired signals. The method also includes, upon detecting the occurrence of the predefined signal feature, modifying a visual feature of at least one of the icon representing the electrode and the icon representing the catheter on the display screen.

As another example, U.S. Patent No. 10,582,872 describes a method and system for visualization of electrophysiology information sensed by electrodes on a catheter. The method includes recording times of electrode signal acquisition, designating a reference electrode signal acquisition, assigning a relative time to each recorded time of electrode signal acquisition relative to the reference electrode signal acquisition, identifying the electrodes with signal acquisition, correlating assigned relative times to identified electrodes to generate a sequence of electrode signal acquisitions, and generating a visual representation of the sequence of electrode signal acquisitions generating a visual representation with a graphical image of the electrodes, wherein individual electrodes are visually marked to represent the sequence of electrode signal acquisitions.

European Patent Application Publication No. EP2662049 describes a method for performing a medical procedure including bringing a probe into contact with an organ in a body of a patient. A map of the organ is displayed, and the location of the probe relative to the map is tracked. A therapy is applied via the probe at multiple tissue sites in the organ with which the probe is brought into contact. Stability of the contact between the probe and the tissue sites is assessed while applying the therapy. The map is automatically marked, responsively to the assessed stability, to indicate the tissue sites at which the therapy was applied. Typically, the system marks sites on the map that satisfy a certain stability criterion, which may be defined by the system operator.

US Patent Application Publication No. 2019/269367 describes transducer-based systems and methods which may be configured to display a graphical representation of a transducer-based device, the graphical representation including graphical elements corresponding to transducers of the transducer-based device, and also including between graphical elements respectively associated with a set of the transducers and respectively associated with a region of space between the transducers of the transducer-based device. Selection of graphical elements and/or between graphical elements can cause activation of the set of transducers associated with the selected elements. Transducer activation characteristics, such as initiation time, activation duration, activation sequence, and energy delivery characteristics, can vary based on numerous factors. Visual characteristics of graphical elements and between graphical elements can change based on an activation-status of the corresponding transducers. Activation requests for a set of transducers can be denied if it is determined that a transducer in the set of transducers is unacceptable for activation.

European Patnet Application Publication No. EP3723098 describes a system including a memory and a processor. The memory is configured to store a definition of a cardiac pacing protocol. The processor is configured to (a) receive the stored definition of the cardiac pacing protocol, (b) in accordance with the pacing protocol, to automatically pace from an intracardiac location and to acquire respective sensed ECG signals, (c) based on one or more prespecified criteria for validity of the sensed ECG data, automatically accept or reject the sensed ECG signals, (d) based on one or more prespecified criteria for identification of an arrhythmia, identify the intracardiac location as an arrhythmogenic focus or pathway, (e) overlay the identified intracardiac location an electrophysiological (EP) map, and (f) subsequently identify or reject a new intracardiac location as an arrhythmogenic focus or pathway and overlay the new location on the EP map when pacing again from the new intracardiac location.

US Patent Application Publication No. 2018/296108 describes systems for facilitating processing of cardiac information based on sensed electrical signals including a processing unit configured to receive a set of electrical signals; receive an indication of a measurement location corresponding to each electrical signal of the set of electrical signals; and generate, based on at least one of an annotation waveform corresponding to each electrical signal of the set of electrical signals and a set of annotation mapping values, an annotation histogram.

### SUMMARY

Embodiments of the present invention that are described hereinbelow provide improved systems for mapping of EP parameters.

There is therefore provided, in accordance with an embodiment of the invention, a system for electrophysiological measurement, including a probe having a distal end configured for insertion into a body cavity of a patient and including electrodes that are disposed along the distal end and are configured to contact tissue at multiple locations within the body cavity while an operator manipulates the probe. The system includes a display and a processor, which is configured to acquire electrophysiological (EP) signals from the electrodes within the body cavity, to apply one or more filtering criteria to the EP signals in order to select a first set of the EP signals while rejecting a second set of the EP signals, to render an image to the display based on the EP signals in the first set, and to output to the operator an indication of a reason for the rejection of the EP signals in the second set. The processor is configured to output the indication with respect to a rejection of the signals by a given filtering criterion only when a percentage of the EP signals that are rejected by the given filtering criterion is greater than a predefined threshold.

In some embodiments, the probe includes a catheter, and the distal end is configured for insertion into a chamber of the heart. In one such embodiment, the processor is configured to track a position of the distal end of the catheter within the chamber and to render to the display an electroanatomical map of the chamber based on the EP signals in the first set. In a disclosed embodiment, the distal end of the catheter includes multiple flexible spines along which the electrodes are disposed.

Typically, the processor is configured to present the indication of the reason for the rejection on the display in a form selected from a group consisting of a textual output and a graphical icon.

In a disclosed embodiment, at least one of the filtering criteria applies to a proximity of the electrodes relative to a wall of the body cavity, and the processor is configured to reject the EP signals in the second set responsively to the at least one of the filtering criteria when a distance between the location of the electrodes and the wall is greater than a given threshold and to indicate to the operator that the distal end of the probe should be brought closer to the wall of the body cavity.

Additionally or alternatively, when the body cavity includes a chamber of a heart of the patient, at least one of the filtering criteria applies to a cycle length of the heart while the processor acquires the EP signals, and the processor is configured to reject the EP signals in the second set responsively to the at least one of the filtering criteria when the cycle length changes during acquisition of the EP signals by more than a given threshold and to indicate to the operator that the EP signals in the second set were rejected because of a change in the cycle length during acquisition of the EP signals.

Further additionally or alternatively, at least one of the filtering criteria applies to a stability of the electrodes relative to a wall of the body cavity, and the processor is configured to reject the EP signals in the second set responsively to the at least one of the filtering criteria when the electrodes move by more than a maximal distance during acquisition of the EP signals and to indicate to the operator to stabilize a manipulation of the probe during the acquisition of the EP signals.

In some embodiments, at least one of the filtering criteria applies to a level of voltage measured by the electrodes, and the processor is configured to reject the EP signals in the second set responsively to the at least one of the filtering criteria and to indicate to the operator that the voltage of the EP signals in the second set was below a certain minimum.

In yet another embodiment, at least one of the filtering criteria applies to a density of the locations at which the EP signals are acquired, and the processor is configured to reject the EP signals in the second set responsively to the at least one of the filtering criteria and to indicate to the operator that the electrodes acquiring the EP signals in the second set were in a region of the body cavity in which a sufficient number of the EP signals was already acquired.

### BRIEF DESCRIPTION OF THE DRAWINGS

The present invention will be more fully understood from the following detailed description of the embodiments thereof, taken together with the drawings in which:
Fig. 1 is a schematic pictorial illustration of a system for electroanatomical mapping, in accordance with an embodiment of the present invention;
Fig. 2 is a schematic illustration of a graphical user interface (GUI) of an electroanatomical mapping system, in accordance with the present disclosure; and and
Fig. 3 is a flow chart that schematically illustrates a method for electroanatomical mapping with automated operator guidance.

### DETAILED DESCRIPTION OF EMBODIMENTS

### OVERVIEW

To produce an accurate electroanatomical map of a heart chamber, the mapping system typically acquires electrical signals from hundreds or even thousands of different points along the wall of the chamber. To reduce the time needed to acquire this large volume of data, mapping systems commonly use catheters having many electrodes at their distal ends, which are capable of sensing respective signals simultaneously at respective locations within the heart chamber. Furthermore, the system may acquire signals from the electrodes in a continuous mode, meaning that the signals received from the electrodes are automatically sampled and recorded continually as the operator moves the distal end of the catheter through the heart chamber.

This sort of continuous-mode mapping can give rise to many spurious measurements, i.e., signals acquired from one or more of the electrodes that do not accurately reflect the actual electrical activity in the myocardium. Such spurious measurements can occur, for example, when the electrode from which a signal was acquired was not actually in contact with the myocardium, or when the electrode was moving too rapidly across the myocardium to make a stable measurement from a well-defined location. It is desirable under such circumstances that the mapping console automatically filter the electrical signals and discard the spurious results, which might otherwise corrupt the map of the heart chamber. Methods and criteria for performing this sort of filtering are described, for example, in U.S. Patent Application No. 16/995,036, filed August 17, 2020, which is assigned to the assignee of the present patent application.

As a result of filtering the signals in this manner, however, many of the signals acquired by the catheter may be discarded, with the result that the rate of acquisition of map data is reduced, and the length of time needed to complete the mapping procedure is increased. The operator of the system may not understand the problems that gave rise to signal rejection and may thus be unable to remedy these problems. In existing systems, the operator may not even be aware that a large fraction of the acquired data has been discarded.

Embodiments of the present invention that are described herein address these problems by automatically providing guidance to the operator of an electroanatomical mapping system. The guidance indicates to the operator why many signals have been rejected and can thus help the operator in improving his or her mapping technique. For example, the operator may use the guidance that is provided in this manner in learning to manipulate the catheter so that the electrodes maintain good contact with the myocardium while sliding smoothly and not too quickly over the appropriate areas of the heart wall.

The disclosed embodiments provide a system for electrophysiological measurement comprising a probe having a distal end configured for insertion into a body cavity of a patient, such as a catheter for insertion into a chamber of the heart, with electrodes disposed along the distal end of the probe. The operator manipulates the probe so that the electrodes contact tissue at multiple locations within the body cavity. A processor connected to the probe acquires electrophysiological (EP) signals from the electrodes within the body cavity and applies filtering criteria to the EP signals. The processor selects a set of the EP signals that satisfy the filtering criteria and uses these signals in rendering an image, such as an electroanatomical map, to a display.

The processor rejects the EP signals that fail to satisfy the filtering criteria. The processor outputs to the operator an indication of the predominant reason or reasons for the rejection of these EP signals, for example in the form of a graphical icon and/or text on the display. The processor is configured to output the indication with respect to a rejection of the signals by a given filtering criterion only when a percentage of the EP signals that are rejected by the given filtering criterion is greater than a predefined threshold. In this way, the processor gives the operator insights into problems in his or her data acquisition techniques, and thus guides the operator in overcoming these problems in order to acquire EP data with greater efficiency and reliability.

### SYSTEM DESCRIPTION

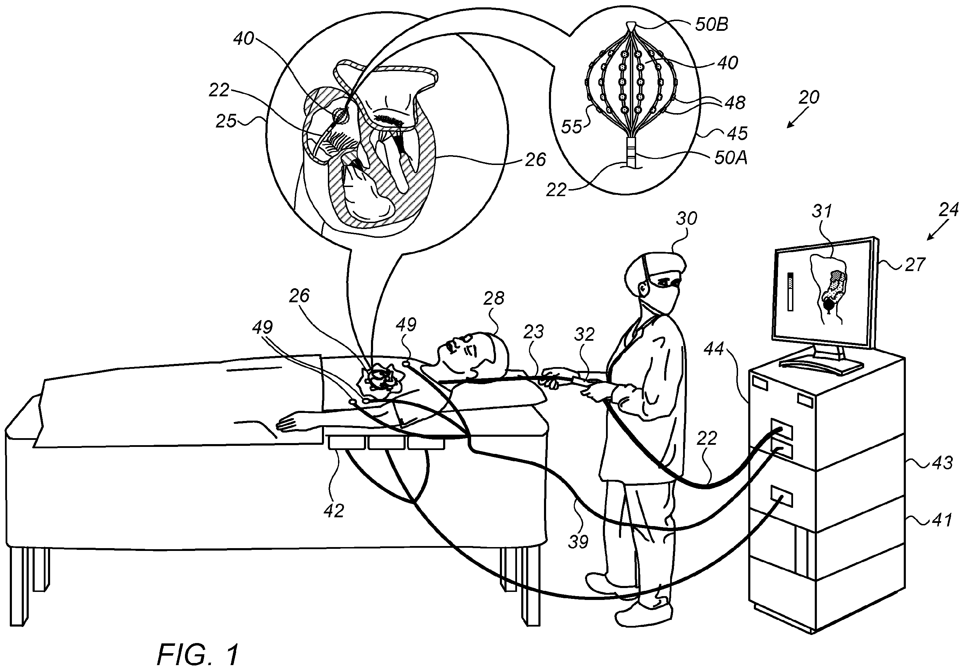

Fig. 1 is a schematic pictorial illustration of a system 20 for mapping an EP parameter in a heart 26 of a patient 28, in accordance with an embodiment of the present invention. The embodiment shown in the current figure and subsequent figures refers to an example of acquiring EP signals from a chamber of heart 26. In alternative embodiments, the values of EP parameters may be acquired using other sorts of mapping apparatus, not only from within the heart, but also from other organs and tissue, as will be apparent to those skilled in the art after reading the present description.

An operator 30, such as a physician, navigates a catheter 22 to a target location in heart 26 of patient 28, by manipulating a shaft 23 of the catheter, using a manipulator 32 near the proximal end of the catheter. In the pictured example, catheter 22 comprises a basket assembly 40 at its distal end, as shown in an inset 45, but alternatively other types of catheters may be used, as are known in the art. As seen in an inset 25, operator 30 manipulates catheter 22 to perform electroanatomical mapping of a chamber of heart 26. EP signals are acquired from the myocardial tissue by bringing electrodes 48 on basket assembly 40 into contact with the tissue within the heart, as further detailed below.

Basket assembly 40 is inserted into heart 26 in a collapsed configuration, for example through a sheath (not shown), and only after the catheter exits the sheath does the basket expand to its intended functional shape, as shown in inset 45. By containing basket assembly 40 in a collapsed configuration, the sheath also serves to minimize vascular trauma along the way to the target location.

For purposes of position tracking, basket assembly 40 incorporates a magnetic sensor 50A, seen in inset 45, at the distal end of catheter 22 (i.e., at the proximal end of the basket assembly). Typically, although not necessarily, sensor 50A is a Triple-Axis Sensor (TAS), comprising three miniature coils oriented in different directions. In the pictured embodiment, a second magnetic sensor 50B is incorporated in the distal end of basket assembly 40. Sensor 50B may be a Single-Axis Sensor (SAS) or a Triple-Axis Sensor (TAS), for example. Alternatively, catheter 22 may comprise other sorts of magnetic sensors, at these or other locations. Alternatively or additionally, the catheter may comprise other sorts of position sensors, such as impedance-based or ultrasonic position sensors, as are known in the art.

Basket assembly 40 comprises multiple expandable spines 55, which are mechanically flexible. Multiple electrodes 48 are fixed to each spine 55, for a total of, for example, 120 electrodes. Electrodes 48 are configured to touch the tissue within heart 26 for the purpose of sensing EP signals, i.e., intracardiac electrogram signals in the pictured example. Magnetic sensors 50A and 50B and electrodes 48 are connected by wires (not shown) running through shaft 22 to processing circuits in a console 24.

Alternatively, system 20 may comprise other types of catheters, with other sorts of electrode arrays, such as an inflatable balloon catheter with electrodes 48 on its outer surface, or a catheter having one or more flexible arms or having a curved "lasso" at its distal end.

System 20 comprises a position-tracking sub-system 43 in console 24 for finding the position and orientation of basket assembly 40, and thereby identifying the locations of electrodes 48. Patient 28 is placed in a magnetic field generated by a pad containing magnetic field generator coils 42, which are driven by position-tracking sub-system 43. The magnetic fields generated by coils 42 give rise to electrical signals in sensors 50A and 50B, which are indicative of the position and orientation of the sensors. The signals from sensors 50A and 50B are transmitted back to position-tracking sub-system 43, which converts the signals to corresponding digital inputs to a processor 41. Processor 41 uses these inputs to compute the position and orientation of basket assembly 40 and thus to find the respective location coordinates of each of electrodes 48.

Methods of position and orientation sensing using external magnetic fields and magnetic sensors, such as sensors 50A and 50B, are implemented in various medical applications, for example, in the CARTO^{®} system, available from Biosense Webster, Inc. (Irvine, California). Such methods are described in detail in U.S. Patent Nos. 5, 391, 199, 6, 690, 963, 6, 484, 118, 6, 239, 724, 6, 618, 612 and 6,332,089, in PCT Patent Publication WO 96/05768, and in U.S. Patent Application Publication Nos. 2002/0065455 A1, 2003/0120150 A1 and 2004/0068178 A1.

Alternatively or additionally, as noted above, system 20 may use other methods of position sensing to find the locations of electrodes 48. For example, processor 41 may map the locations of electrodes 48 by measuring impedances between electrodes 48 and body-surface electrodes 49, which are placed on the chest of patient 28 and connected to console 24 by leads 39.

Processor 41 additionally receives EP signals from electrodes 48 on basket assembly 40 via front-end circuits 44. These circuits apply analog and/or digital filters and amplifiers to the signals under the control of the processor. Processor 41 uses the information contained in these EP signals together with the coordinates provided by magnetic sensors 50A and 50B in constructing an electroanatomical map 31 of the chamber of heart 26 in which basket assembly 40 is located, such as a map showing the voltage levels or local activation time (LAT) of the EP signals as a function of location along the chamber walls. During and/or following the procedure, processor 41 renders electroanatomical map 31 to a display 27.

In choosing the EP signals to include in the map, processor 41 applies filtering criteria, while rejecting the signals that do not meet these criteria. These criteria may be fixed, or they may be adjusted by an operator of system 20, for example as described in the above-mentioned U.S. Patent Application No. 16/995,036. Examples of such criteria and their application are presented hereinbelow. When a percentage of the EP signals that are rejected by a given filtering criterion is greater than a predefined threshold, processor 41 outputs an indication of the reason for rejection, for example in graphical and/or textual form, to display 27.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 41 runs a dedicated algorithm that enables the processor to perform the disclosed steps of data acquisition, mapping, and operator guidance, as described below.

The example illustration shown in Fig. 1 is chosen purely for the sake of conceptual clarity. Fig. 1 shows only elements related to the disclosed techniques for the sake of simplicity and clarity. System 20 typically comprises additional modules and elements that are not directly related to the disclosed techniques, and thus are intentionally omitted from Fig. 1 and from the corresponding description.

### EP SIGNAL FILTERING AND OPERATOR GUIDANCE

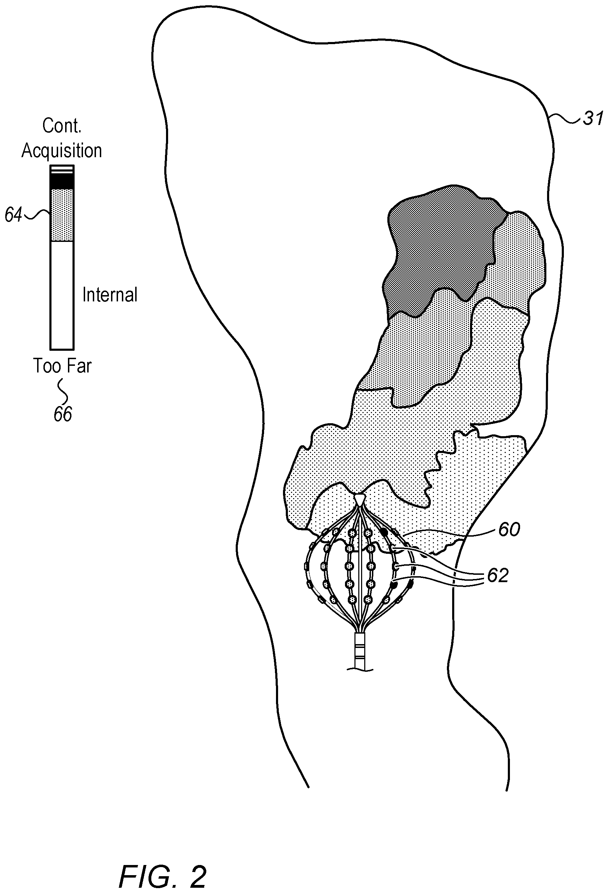

Fig. 2 is a schematic illustration of a graphical user interface (GUI), which is presented on display 27 of system 20. Processor 41 renders electroanatomical map 31 of a chamber of heart 26 to display 27, based on the EP signals acquired by electrodes 48 and the position information provided by position-tracking sub-system 43. Processor 41 superimposes a basket icon 60 on map 31, showing the current position of basket assembly 40 at the distal end of catheter 22 within the heart chamber, with icon segments 62 corresponding to electrodes 48.

Map 31 shows the three-dimensional (3D) form of the inner wall of a heart chamber into which the distal end of catheter 22 is inserted, with coloring to indicate the EP parameters sensed by electrodes 48. The 3D form of the chamber wall is reconstructed, for example, by a process of fast anatomical mapping (FAM), which locates the outer bounds of a point cloud formed by the position coordinates of basket assembly 40 as it moves through the heart chamber. FAM techniques that can be used in this context are described, for example, in U.S. Patent No. 10,420,612 and in U.S. Patent Application Publication No. 2019/0200901. The coloring may indicate the LAT or voltage level measured by electrodes 48 at each location along the reconstructed form of the chamber wall.

In the present embodiment, processor 41 acquires the EP signals and position coordinates in continuous mode, as the operator manipulates catheter 22 within the heart 26, and updates map 31 continually based on the acquired information. In other words, the processor does not wait for a specific operator input to capture data from catheter 22, but rather samples the EP signals and position data autonomously at regular intervals. As noted earlier and described in greater detail in the above-mentioned U.S. Patent Application No. 16/995,036, processor 41 applies filtering criteria to the EP signals in order to select the set of signals to be incorporated in map 31 and reject the signals that do not satisfy the criteria.

Based on the results of application of the filtering criteria, processor 41 also renders an operator guidance icon 64 and/or guidance text 66 to display 27. Alternatively or additionally, this guidance information may be conveyed by other means, such as an audio output from console 24. Icon 64 and text 66 indicate the reason or reasons for rejection of a substantial fraction of the EP signals by the filtering criteria and thus guide the operator in achieving more effective signal acquisition. Icon 64 may be color-coded, for example, according to the reasons for rejection. Optionally, icon segments 62 may be color-coded in a similar fashion to show the locations of electrodes 48 whose output signals were rejected for the corresponding reasons.

By way of example, but not limitation, the reasons for rejection of EP signals and the corresponding operator indications and guidance can include the following:
- Electrodes not in proximity to the wall of the heart chamber, for example too far from the surface of the wall that has been reconstructed by the FAM algorithm. This is the situation that is illustrated in Fig. 2, with icon 64 showing a large fraction of the electrodes to be "internal," i.e., inside the volume of the chamber. Text 66 tells the operator that basket assembly 40 is "too far" from the chamber wall.
- Cycle length has changed during acquisition. For accurate mapping, it is important that the length of the heart cycle remain within predefined bounds during the process of data acquisition. When the cycle length (i.e., the heart rate) changes by more than a given threshold, processor 41 will reject the EP signals and update icon 64 and text 66 accordingly. The change in cycle length may be spontaneous, as the result of physiological processes, or it may be caused by the operator, for example by pacing the patient's heart.
- Catheter was unstable during acquisition. Processor 41 senses the extent and rate of motion of basket assembly 40 during acquisition of EP signals. When the basket assembly moves by more than a certain maximal distance during acquisition of an EP sample or set of samples, the processor will reject the signals. In this case, processor 41 will update icon 64 and text 66 to indicate that basket assembly 40 was moving too quickly during acquisition, thus indicating to the operator that the manipulation of catheter 22 should be stabilized.
- Voltage too low. Processor 41 filters the EP signals by voltage level and will incorporate into map 31 only the signals whose voltage was above a certain minimum. When many or all of the signals in a given area are rejected for failure to satisfy this criterion, processor 41 will update icon 64 and text 66 to indicate that the signal voltages were below the minimum. The indication may suggest to the operator that this area of the heart wall is characterized by low voltage, meaning that further mapping in this area will be unproductive.
- Acquisition completed in this area of the chamber. Processor 41 checks the density of the locations at which EP signals have been acquired, and filters out further data in areas in which a sufficient number of EP signals has already been acquired. In this case, icon 64 and text 66 will indicate to the operator that catheter 22 should be moved to another area of the heart chamber.
The above criteria and operator indications are presented by way of example, and other sorts of guidance may be derived from the EP signals and location data and presented to the operator in similar fashion.

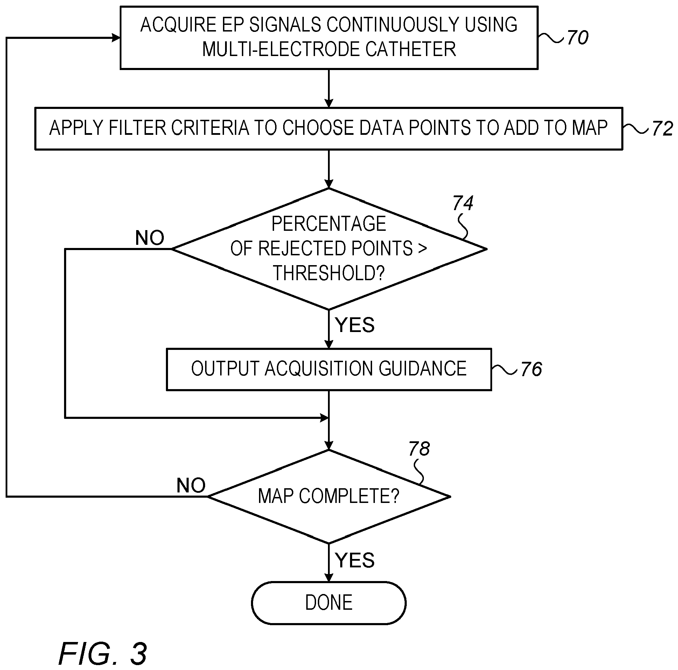

Fig. 3 is a flow chart that schematically illustrates a method for electroanatomical mapping with automated operator guidance. The process is described here, for the sake of concreteness and clarity, with reference to the elements of system 20 (Fig. 1). Alternatively, the principles of this method may be applied, *mutatis mutandis,* in other system configurations used for EP signal acquisition, in the heart as well as in other body cavities.

Operator 30 inserts catheter 22 into heart 26, so that basket assembly 40 opens and electrodes 48 sense EP signals within the heart, at a signal acquisition step 70. Processor 41 collects the signals continuously, as explained above, while operator 30 manipulates the catheter within the heart. The processor applies filter criteria to the EP signals, such as the criteria described above, in order to select the set of the signals that satisfy the criteria, at a signal selection step 72. The processor extracts the parameters of interest from these signals, such as the voltage or LAT, and uses these parameters together with the coordinates of the electrodes that acquired the signals in constructing a map of the heart chamber. The processor continues to construct and add to the map continually as the operator moves the distal end of the catheter along the wall of the heart chamber.

Over each period of signal acquisition, processor 41 evaluates the percentage of the EP signals that are rejected by the filtering criteria, at a rejection assessment step 74. When the percentage of the signals rejected by a given filtering criterion exceeds a predefined threshold, the processor outputs an indication of the reason for rejection, at an acquisition guidance step 76. The guidance at this step typically takes the form of icon 64 and/or text 66, as shown in Fig. 2, but it may alternatively be provided by other means.

Whether or not the acquisition guidance is provided at a given stage in the mapping process, acquisition of signals and addition of data points to map 31 continues until mapping of the chamber is completed, at a map completion step 78.

Although the embodiments described above relate specifically to systems for electroanatomical mapping of the heart, the principles of the present invention may similarly be applied, *mutatis mutandis,* in other EP mapping and diagnostic procedures, as well as in diagnostic systems of other sorts in which large amounts of data are acquired within the body under the control of a human operator. It will thus be appreciated that the embodiments described above are cited by way of example, and that the present invention is not limited to what has been particularly shown and described hereinabove. Rather, the scope of the present invention is defined by the appended claims and may include both combinations and subcombinations of the various features described hereinabove, as well as variations and modifications thereof which would occur to persons skilled in the art upon reading the foregoing description and which are not disclosed in the prior art.

## Claims

1. A system (20) for electrophysiological measurement, comprising:
a probe (22) having a distal end configured for insertion into a body cavity of a patient and comprising electrodes (48) that are disposed along the distal end and are configured to contact tissue at multiple locations within the body cavity while an operator manipulates the probe (22);
a display (27); and
a processor (41) configured to acquire electrophysiological (EP) signals from the electrodes (48) within the body cavity, to apply one or more filtering criteria to the EP signals in order to select a first set of the EP signals while rejecting a second set of the EP signals, to render an image to the display (27) based on the EP signals in the first set, and to output to the operator an indication of a reason for the rejection of the EP signals in the second set, wherein the processor (41) is configured to output the indication with respect to a rejection of the signals by a given filtering criterion only when a percentage of the EP signals that are rejected by the given filtering criterion is greater than a predefined threshold.

2. The system according to claim 1, wherein the probe comprises a catheter (22), and the distal end is configured for insertion into a chamber of the heart.

3. The system according to claim 2, wherein the processor (41) is configured to track a position of the distal end of the catheter (22) within the chamber and to render to the display an electroanatomical map (31) of the chamber based on the EP signals in the first set.

4. The system according to claim 2, wherein the distal end of the catheter (22) comprises multiple flexible spines along which the electrodes (48) are disposed.

5. The system according to claim 1, wherein the processor (41) is configured to present the indication of the reason for the rejection on the display in a form selected from a group consisting of a textual output (66) and a graphical icon (64).

6. The system according to claim 1, wherein at least one of the filtering criteria applies to a proximity of the electrodes (48) relative to a wall of the body cavity, and wherein the processor is configured to reject the EP signals in the second set responsively to the at least one of the filtering criteria when a distance between the location of the electrodes (48) and the wall is greater than a given threshold and to indicate to the operator that the distal end of the probe should be brought closer to the wall of the body cavity.

7. The system according to claim 1, wherein the body cavity comprises a chamber of a heart of the patient, and wherein at least one of the filtering criteria applies to a cycle length of the heart while the processor acquires the EP signals, and wherein the processor (41) is configured to reject the EP signals in the second set responsively to the at least one of the filtering criteria when the cycle length changes during acquisition of the EP signals by more than a given threshold and to indicate to the operator that the EP signals in the second set were rejected because of a change in the cycle length during acquisition of the EP signals.

8. The system according to claim 1, wherein at least one of the filtering criteria applies to a stability of the electrodes (48) relative to a wall of the body cavity, and wherein the processor (41) is configured to reject the EP signals in the second set responsively to the at least one of the filtering criteria when the electrodes move by more than a maximal distance during acquisition of the EP signals and to indicate to the operator to stabilize a manipulation of the probe during the acquisition of the EP signals.

9. The system according to claim 1, wherein at least one of the filtering criteria applies to a level of voltage measured by the electrodes (48), and wherein the processor (41) is configured to reject the EP signals in the second set responsively to the at least one of the filtering criteria and to indicate to the operator that the voltage of the EP signals in the second set was below a certain minimum.

10. The system according to claim 1, wherein at least one of the filtering criteria applies to a density of the locations at which the EP signals are acquired, and wherein the processor (48) is configured to reject the EP signals in the second set responsively to the at least one of the filtering criteria and to indicate to the operator that the electrodes (48) acquiring the EP signals in the second set were in a region of the body cavity in which a sufficient number of the EP signals was already acquired.

## Patentansprüche

1. System (20) für eine elektrophysiologische Messung, umfassend:
eine Sonde (22), die ein distales Ende aufweist, das für eine Einführung in einen Körperhohlraum eines Patienten konfiguriert ist, und umfassend Elektroden (48), die entlang des distalen Endes angeordnet und konfiguriert ist, um Gewebe an mehreren Stellen innerhalb des Körperhohlraums zu berühren, während ein Bediener die Sonde (22) betätigt;
eine Anzeige (27); und
einen Prozessor (41), der konfiguriert ist, um elektrophysiologische (EP) Signale von den Elektroden (48) innerhalb des Körperhohlraums zu erfassen, ein oder mehrere Filterkriterien auf die EP-Signale anzuwenden, um einen ersten Satz der EP-Signale auszuwählen während ein zweiter Satz der EP-Signale abgelehnt wird, ein Bild auf der Anzeige (27) basierend auf den EP-Signalen in dem ersten Satz darzustellen und dem Bediener einen Hinweis auf einen Grund für die Ablehnung der EP-Signale in dem zweiten Satz auszugeben, wobei der Prozessor (41) konfiguriert ist, um den Hinweis in Bezug auf eine Ablehnung der Signale durch ein gegebenes Filterkriterium nur dann auszugeben, wenn ein Prozentsatz der EP-Signale, die durch das gegebene Filterkriterium abgelehnt werden, größer als ein vordefinierter Schwellenwert ist.

2. System nach Anspruch 1, wobei die Sonde einen Katheter (22) umfasst und das distale Ende für die Einführung in eine Kammer des Herzens konfiguriert ist.

3. System nach Anspruch 2, wobei der Prozessor (41) konfiguriert ist, um eine Position des distalen Endes des Katheters (22) innerhalb der Kammer zu verfolgen und eine elektroanatomische Karte (31) der Kammer basierend auf den EP-Signalen in dem ersten Satz auf der Anzeige darzustellen.

4. System nach Anspruch 2, wobei das distale Ende des Katheters (22) mehrere flexible Stacheln umfasst, entlang derer die Elektroden (48) angeordnet sind.

5. System nach Anspruch 1, wobei der Prozessor (41) konfiguriert ist, um den Hinweis auf den Grund für die Ablehnung auf der Anzeige in einer Form zu präsentieren, die aus einer Gruppe ausgewählt ist, bestehend aus einer Textausgabe (66) und einem grafischen Symbol (64).

6. System nach Anspruch 1, wobei mindestens eines der Filterkriterien auf eine Nähe der Elektroden (48) relativ zu einer Wand des Körperhohlraums zutrifft, und wobei der Prozessor konfiguriert ist, um die EP-Signale in dem zweiten Satz als Reaktion auf das mindestens eine der Filterkriterien abzulehnen, wenn ein Abstand zwischen der Stelle der Elektroden (48) und der Wand größer als ein gegebener Schwellenwert ist, und den Bediener darauf hinzuweisen, dass das distale Ende der Sonde näher an die Wand des Körperhohlraums gebracht werden sollte.

7. System nach Anspruch 1, wobei der Körperhohlraum eine Kammer eines Herzens des Patienten umfasst, und wobei mindestens eines der Filterkriterien auf eine Zykluslänge des Herzens zutrifft, während der Prozessor die EP-Signale erfasst, und wobei der Prozessor (41) konfiguriert ist, um die EP-Signale in dem zweiten Satz als Reaktion auf das mindestens eine der Filterkriterien abzulehnen, wenn sich die Zykluslänge während der Erfassung der EP-Signale um mehr als einen gegebenen Schwellenwert ändert, und den Bediener darauf hinzuweisen, dass die EP-Signale in dem zweiten Satz aufgrund einer Änderung in der Zykluslänge während der Erfassung der EP-Signale abgelehnt wurden.

8. System nach Anspruch 1, wobei mindestens eines der Filterkriterien auf eine Stabilität der Elektroden (48) relativ zu einer Wand des Körperhohlraums zutrifft, und wobei der Prozessor (41) konfiguriert ist, um die EP-Signale in dem zweiten Satz als Reaktion auf das mindestens eine der Filterkriterien abzulehnen, wenn sich die Elektroden während der Erfassung der EP-Signale um mehr als einen maximalen Abstand bewegen, und den Bediener darauf hinzuweisen, während der Erfassung der EP-Signale eine Betätigung der Sonde zu stabilisieren.

9. System nach Anspruch 1, wobei mindestens eines der Filterkriterien auf einen Spannungspegel zutrifft, der durch die Elektroden (48) gemessen wird, und wobei der Prozessor (41) konfiguriert ist, um die EP-Signale in dem zweiten Satz als Reaktion auf das mindestens eine der Filterkriterien abzulehnen und den Bediener darauf hinzuweisen, dass die Spannung der EP-Signale in dem zweiten Satz unter einem bestimmten Minimum lag.

10. System nach Anspruch 1, wobei mindestens eines der Filterkriterien auf eine Dichte der Stellen zutrifft, an denen die EP-Signale erfasst werden, und wobei der Prozessor (48) konfiguriert ist, um die EP-Signale in dem zweiten Satz als Reaktion auf mindestens das eine der Filterkriterien abzulehnen und den Bediener darauf hinzuweisen, dass sich die Elektroden (48), die die EP-Signale in dem zweiten Satz erfassen, in einem Bereich des Körperhohlraums befanden, in dem bereits eine ausreichende Anzahl der EP-Signale erfasst wurde.

## Revendications

1. Système (20) de mesure électrophysiologique, comprenant :
une sonde (22) ayant une extrémité distale conçue pour être insérée dans une cavité corporelle d'un patient et comprenant des électrodes (48) qui sont disposées le long de l'extrémité distale et sont conçues pour entrer en contact avec le tissu au niveau de plusieurs emplacements de la cavité corporelle pendant qu'un opérateur manipule la sonde (22) ;
un écran (27) ; et
un processeur (41) configuré pour acquérir des signaux électrophysiologiques (EP) à partir des électrodes (48) dans la cavité corporelle, pour appliquer un ou plusieurs critères de filtrage aux signaux EP afin de sélectionner un premier ensemble de signaux EP tout en rejetant un second ensemble des signaux EP, pour restituer une image à l'écran (27) sur la base des signaux EP dans le premier ensemble, et pour fournir en sortie à l'opérateur une indication d'une raison du rejet des signaux EP dans le second ensemble, dans lequel le processeur (41) est configuré pour fournir en sortie l'indication concernant un rejet des signaux par un critère de filtrage donné uniquement lorsqu'un pourcentage des signaux EP qui sont rejetés par le critère de filtrage donné est supérieur à un seuil prédéfini.

2. Système selon la revendication 1, dans lequel la sonde comprend un cathéter (22), et l'extrémité distale est conçue pour être insérée dans une chambre du coeur.

3. Système selon la revendication 2, dans lequel le processeur (41) est configuré pour suivre une position de l'extrémité distale du cathéter (22) dans la chambre et pour restituer à l'écran une carte électroanatomique (31) de la chambre sur la base des signaux EP du premier ensemble.

4. Système selon la revendication 2, dans lequel l'extrémité distale du cathéter (22) comprend plusieurs épines souples le long desquelles sont disposées les électrodes (48).

5. Système selon la revendication 1, dans lequel le processeur (41) est configuré pour présenter l'indication de la raison du rejet sur l'écran sous une forme choisie dans un groupe constitué d'une sortie textuelle (66) et d'une icône graphique (64).

6. Système selon la revendication 1, dans lequel au moins l'un parmi les critères de filtrage s'applique à une proximité des électrodes (48) par rapport à une paroi de la cavité corporelle, et dans lequel le processeur est configuré pour rejeter les signaux EP dans le second ensemble en réponse à l'au moins un parmi les critères de filtrage lorsqu'une distance entre l'emplacement des électrodes (48) et la paroi est supérieure à un seuil donné et pour indiquer à l'opérateur que l'extrémité distale de la sonde doit être rapprochée de la paroi de la cavité corporelle.

7. Système selon la revendication 1, dans lequel la cavité corporelle comprend une chambre d'un coeur du patient, et dans lequel au moins l'un parmi les critères de filtrage s'applique à une longueur de cycle du coeur pendant que le processeur acquiert les signaux EP, et dans lequel le processeur (41) est configuré pour rejeter les signaux EP dans le second ensemble en réponse à l'au moins un parmi les critères de filtrage lorsque la longueur de cycle change pendant l'acquisition des signaux EP de plus d'un seuil donné et pour indiquer à l'opérateur que les signaux EP dans le second ensemble ont été rejetés en raison d'un changement dans la longueur de cycle pendant l'acquisition des signaux EP.

8. Système selon la revendication 1, dans lequel au moins l'un parmi les critères de filtrage s'applique à une stabilité des électrodes (48) par rapport à une paroi de la cavité corporelle, et dans lequel le processeur (41) est configuré pour rejeter les signaux EP dans le second ensemble en réponse à l'au moins un parmi les critères de filtrage lorsque les électrodes se déplacent de plus d'une distance maximale pendant l'acquisition des signaux EP et pour indiquer à l'opérateur de stabiliser une manipulation de la sonde pendant l'acquisition des signaux EP.

9. Système selon la revendication 1, dans lequel au moins l'un parmi les critères de filtrage s'applique à un niveau de tension mesuré par les électrodes (48), et dans lequel le processeur (41) est configuré pour rejeter les signaux EP dans le second ensemble en réponse à l'au moins un parmi les critères de filtrage et pour indiquer à l'opérateur que la tension des signaux EP dans le second ensemble était inférieure à un certain minimum.

10. Système selon la revendication 1, dans lequel au moins l'un parmi les critères de filtrage s'applique à une densité des emplacements au niveau desquels les signaux EP sont acquis, et dans lequel le processeur (48) est configuré pour rejeter les signaux EP dans le second ensemble en réponse à l'au moins un parmi les critères de filtrage et pour indiquer à l'opérateur que les électrodes (48) acquérant les signaux EP dans le second ensemble se trouvaient dans une région de la cavité corporelle dans laquelle un nombre suffisant de signaux EP était déjà acquis.
